# EUROPÄISCHE PATENTSCHRIFT

(11) **EP 1 077 651 B1**
(45) Veröffentlichungstag und Bekanntmachung des Hinweises auf die Patenterteilung: **03.12.2003**
(21) Anmeldenummer: 99904689.9
(22) Anmeldetag: 04.03.1999
(51) Int. Cl.: A61B 17/34, A61B 19/00

(54) **PUNKTIONSVORRICHTUNG FÜR SCHICHTAUFNAHMEVERFAHREN**
PUNCTURING DEVICE FOR TOMOGRAPHY METHODS
DISPOSITIF DE PONCTION DESTINE A LA TOMOGRAPHIE

(30) Priorität: 13.05.1998 CH 106698
(43) Veröffentlichungstag der Anmeldung: 28.02.2001
(73) Patentinhaber: Tomo-Vision GmbH, 3122 Kehrsatz (CH)
(72) Erfinder: JÄGGI, Kurt, CH-3095 Spiegel bei Bern (CH)
(74) Vertreter: BOVARD AG - Patentanwälte
(86) Internationale Anmeldenummer: CH9900103
(87) Internationale Veröffentlichungsnummer: WO99058069

(56) Entgegenhaltungen:
- EP-A- 0 422 396
- EP-A- 0 640 842
- EP-A- 0 832 610
- DE-C- 19 627 314
- US-A- 4 826 487
- US-A- 5 178 146

## Beschreibung

Die Erfindung betrifft eine Punktionsvorrichtung für Schichtaufnahmeverfahren, enthaltend einen Körper, mit der Aufnahmeebene auszurichtende Führungsmittel für eine Punktionsnadel und im Körper angeordnete, längliche Hohlräume, die ein Kontrastmittel enthalten.

Unter Schichtaufnahmeverfahren sollen im Zusammenhang mit der vorliegenden Erfindung in der Medizin verwendete bildgebende Verfahren verstanden werden, insbesondere in Echtzeit arbeitendes MRI (magnetic resonance imaging) und CT (computed tomography).

Durch Schichtaufnahmeverfahren überwachte Punktionen sind mit verschiedenen Schwierigkeiten und Problemen verbunden. Die Platzverhältnisse im Aufnahmegerät sind sehr eng, wodurch die Dimensionen einer Punktionsvorrichtung begrenzt sind. Beim MRI können nicht beliebige Materialien, insbesondere keine ferromagnetischen Werkstoffe in das Magnetfeld gebracht werden. Der Arzt verfolgt die Punktionsnadel auf einem Bildschirm und kann diese nur sehen, solange sie sich mindestens annähernd in der Schnittebene befindet, welche beim MRI durch das Magnetfeld und beim CT durch die Röntgenstrahlen bestimmt ist. So kann es durchaus vorkommen, dass die Punktionsnadel zwar im Bereich der Einstichstelle sichtbar ist, jedoch ihre Spitze nicht, weil sich diese vor oder hinter der Bildebene befindet. Die Schwierigkeiten, die dem Arzt in einer solchen Situation begegnen, sind leicht vorstellbar. Beim Einstechen der Punktionsnadel, insbesondere in der Bauchgegend eines Patienten, kann sich das Gewebe zuerst stark nach innen wölben, wodurch ein anvisiertes zu punktierendes Organ oder ein Tumor seitwärts ausweichen kann.

Es sind verschiedene Vorrichtungen bekannt, die der genauen Positionierung eines Patienten in einem Schichtaufnahmegerät dienen und die somit auch bei Punktionen Verwendung finden können. Mehrere dieser Vorrichtungen arbeiten mit optischer Vermessung von am Körper des Patienten angebrachten Bezugspunkten. Diese Bezugspunkte können auf die Haut gezeichnete oder geklebte oder auch projizierte Markierungen sein. Letztere können beispielsweise mittels eines Laserstrahls erzeugt werden. Allen diesen optischen Vorrichtungen ist gemeinsam, dass sie sehr aufwendig und dadurch teuer sind. Ausserdem können sie aufgrund ihrer Abmessungen und Anordnung an der Schichtaufnahmeeinrichtung den Arzt bei einer Punktion behindern, oder der Arzt kann die Funktion der Positioniervorrichtung stören, wenn er ungewollt die Messstrahlen unterbricht. Zudem ist bei keiner der Vorrichtungen dieser Art eine einfache und präzise Nadelführung vorgesehen.

Die Patentschrift US 4 826 487 betrifft einen Ausrichtknopf für einen Stereotaxie-Stöpsel und ein Verfahren zur Verwendung desselben. Der Stöpsel kann an seinem Aussenumfang ein Gewinde tragen und ist dazu bestimmt, in die Schädeldecke eines Patienten eingesetzt zu werden. Er trägt mehrere, in einem bestimmten Muster angeordnete axiale Bohrungen, von denen eine erste Gruppe als Führungskanülen für chirurgische Instrumente ausgebildet ist. Eine zweite Gruppe von axialen Bohrungen ist als Markerbohrungen ausgebildet, wobei die Bohrungen ein vom jeweiligen Schichtaufnahmeverfahren abhängiges Kontrastmittel enthalten. Für Aufnahmen mit Röntgenstrahlen wird als Kontrastmittel Gold vorgeschlagen und für MRI Aufnahmen Wasser oder Lipide. Nach dem Einsetzten des Stöpsels wird die Kopfhaut darüber gezogen und vernäht, um das Infektionsrisiko möglichst gering zu halten. Der Ausrichtknopf enthält in einem mit dem Muster des Stöpsels identischen Muster angeordnete Kanülen und Marker-Bohrungen. Er wird in einem entsprechenden Aufnahmegerät genau mit dem Stöpsel ausgerichtet und dann an der Kopfhaut angenäht. Mit diesem System ist sowohl eine exakte Ortung der Einstichstelle mittels eines Schichtaufnahmeverfahrens als auch eine genaue Führung eines chirurgischen Instrumentes, beispielsweise einer Punktionsnadel möglich. Jedoch ist dieses System nur für in zeitlichen Abständen wiederholte Eingriffe, insbesondere im Schädel, sinnvoll einsetzbar und eignet sich nicht für einmalige Punktionen. In der Patentschrift ist angegeben, dass der Ausrichtknopf auch alleine verwendet werden kann. Dadurch entfällt aber die Führung der Nadel, so dass zwar die Einstichstelle, nicht aber die Einstichrichtung der Nadel durch den Ausrichtknopf festlegbar ist.

Die Patentschrift US 5 178 146 betrifft ein System zum Ausrichten eines Patienten zur Verwendung von MRI oder anderen bildgebenden Verfahren. Das System enthält insbesondere einen mit Abstand um den zu untersuchenden Bereich des Körpers des Patienten zu legenden quaderförmigen Kasten, in dessen Wänden mit einem Kontrastmittel gefüllte Rohre in einem bestimmten Muster verlegt sind. Als Kontrastmittel für MRI ist beispielsweise eine Gadolinium-Verbindung angegeben und für CT eine Barium-Verbindung. Es sind sowohl in einem Rechteckmuster angeordnete als auch diagonal verlaufende Rohre vorgesehen. Letztere erzeugen im Bild Schatten, die sich in der Bildebene verschieben, wenn der Kasten durch das Feld bewegt wird. An der gegenseitigen Position dieser Schatten kann der Arzt erkennen, ob der Patient die gewünschte Position im Aufnahmegerät einnimmt. Dieses System ist insbesondere dazu bestimmt, mittels MRI erzeugte Bilder mit durch andere Verfahren erzeugten Bildern in Übereinstimmung zu bringen. Dazu ist das Kontrastmittel in den Rohren austauschbar. Ein Anwendungsbeispiel findet sich in der Strahlentherapie, indem beispielsweise ein Tumor zuerst mittels MRI genau lokalisiert und anschliessend mit Röntgenstrahlen bestrahlt wird. Die genaue Position zum Bestrahlen ist dann erreicht, wenn das durch die Rohre auf dem CT-Bild erzeugte Muster mit demjenigen auf den zuvor erzeugten MRI-Bild übereinstimmt. Zwar ist in der Patentschrift erwähnt, dass das System auch für Punktionen Anwendung finden könne, jedoch finden sich keine Hinweise auf eine Nadelführung. Darüber hinaus ist es offensichtlich, dass der genannte Kasten den Arzt beim Punktieren zusätzlich behindern kann.

Es ist eine Aufgabe der vorliegenden Erfindung, eine Punktionsvorrichtung zu schaffen, die einfach und kostengünstig hergestellt sowie einfach, sicher und zuverlässig betrieben werden kann. Eine weitere Aufgabe der Erfindung besteht darin, eine Punktionsvorrichtung vorzuschlagen, deren Abmessungen verhältnismässig gering sind und die daher auch bei den im Aufnahmegerät herrschenden engen Platzverhältnissen einsetzbar ist. Eine weitere Aufgabe der Erfindung besteht in der Schaffung einer Punktionsvorrichtung, die sich aus Werkstoffen herstellen lässt, die beim betreffenden Schichtaufnahmeverfahren keine Störungen verursachen. Ferner hat die Erfindung zum Ziel, dem Arzt eine Punktionsvorrichtung zur Verfügung zu stellen, mit der die Punktionsnadel exakt mit der Bildebene ausgerichtet werden kann, so dass diese jederzeit von der Einstichstelle bis zur Spitze auf dem Bildschirm beobachtet werden kann. Schliesslich soll mit der erfindungsgemässen Punktionsvorrichtung der betreffende Körperbereich des Patienten vor dem Einstechen der Punktionsnadel komprimiert werden können, so dass die Einbuchtung dieses Bereichs und dadurch ein Ausweichen des zu punktierenden Organs oder Tumors beim Einstechen minimiert wird.

Diese Aufgaben werden durch eine Punktionsvorrichtung der eingangs genannten Art gelöst, die dadurch gekennzeichnet ist, dass der Körper im wesentlichen die Form eines Quaders hat, wobei dessen zur Längsachse parallele Grundfläche zum Aufsetzen auf die Haut eines Patienten bestimmt ist, dass die Achse der Führungsmittel die Grundfläche in einem rechten Winkel schneidet und dass mindestens zwei der länglichen Hohlräume rechtwinklig zur Längsachse, aber nicht parallel zu den Führungsmitteln und nicht parallel zueinander angeordnet sind.

Eine weitere Aufgabe der Erfindung besteht darin, eine Punktionsvorrichtung vorzuschlagen, die nach dem Einführen der Punktionsnadel in den Körper eines Patienten entfernt werden kann, ohne dass dazu die Punktionsnadel herausgezogen werden muss. Diese Aufgabe wird durch eine Punktionsvorrichtung gelöst, welche die Merkmale des Anspruchs 7 aufweist.

Weitere besondere Ausführungsarten der erfindungsgemässen Punktionsvorrichtung sind in den abhängigen Ansprüchen umschrieben.

Die Erfindung wird nachstehend unter Bezugnahme auf in den Zeichnungen dargestellte Ausführungsarten näher beschrieben. Es zeigt
- Figur 1: einen vergrössert dargestellten Aufriss eines Ausführungsbeispiels der erfindungsgemässen Punktionsvorrichtung,
- Figur 2: einen Grundriss des Ausführungsbeispiels gemäss Figur 1,
- Figur 3: einen Seitenriss des Ausführungsbeispiels gemäss Figur 1,
- Figur 4A: eine perspektivische Darstellung einer korrekt ausgerichteten Punktionsvorrichtung gemäss Figur 1,
- Figur 4B: eine schematisch dargestellte Schichtaufnahme der nach Figur 4A ausgerichteten Vorrichtung,
- Figur 5A: eine perspektivische Darstellung einer Punktionsvorrichtung gemäss Figur 1, die gegenüber der Bildebene parallel verschoben ist,
- Figur 5B: eine schematisch dargestellte Schichtaufnahme der nach Figur 5A angeordneten Vorrichtung,
- Figur 6A: eine perspektivische Darstellung einer Punktionsvorrichtung gemäss Figur 1, die um eine vertikale Achse verdreht in der Bildebene angeordnet ist,
- Figur 6B: eine schematisch dargestellte Schichtaufnahme der nach Figur 6A angeordneten Vorrichtung,
- Figur 7A: eine perspektivische Darstellung einer Punktionsvorrichtung gemäss Figur 1, die um eine horizontale Achse verdreht in der Bildebene angeordnet ist,
- Figur 7B: eine schematisch dargestellte Schichtaufnahme der nach Figur 7A angeordneten Vorrichtung,
- Figur 8A: eine perspektivische Darstellung einer Punktionsvorrichtung gemäss Figur 1, die um eine horizontale und eine vertikale Achse verdreht in der Bildebene angeordnet ist,
- Figur 8B: eine schematisch dargestellte Schichtaufnahme der nach Figur 8A angeordneten Vorrichtung,
- Figur 9: eine perspektivische Ansicht eines zweiten Ausführungsbeispiels der erfindungsgemässen Punktionsvorrichtung, bestehend aus zwei identischen, durch Spritzgiessen hergestellten Teilen, die gerade zusammengefügt werden,
- Figur 10: das Ausführungsbeispiel gemäss Figur 9 in fertig montiertem, gebrauchsbereitem Zustand,
- Figur 11: eine schematisch dargestellte Schichtaufnahme der Vorrichtung nach Figur 10 bei korrekter Ausrichtung,
- Figur 12: das Ausführungsbeispiel gemäss Figur 9 nach dem Gebrauch in geteiltem Zustand und
- Figur 13: eine alternative Ausführungsart der erfindungsgemässen Punktionsvorrichtung.

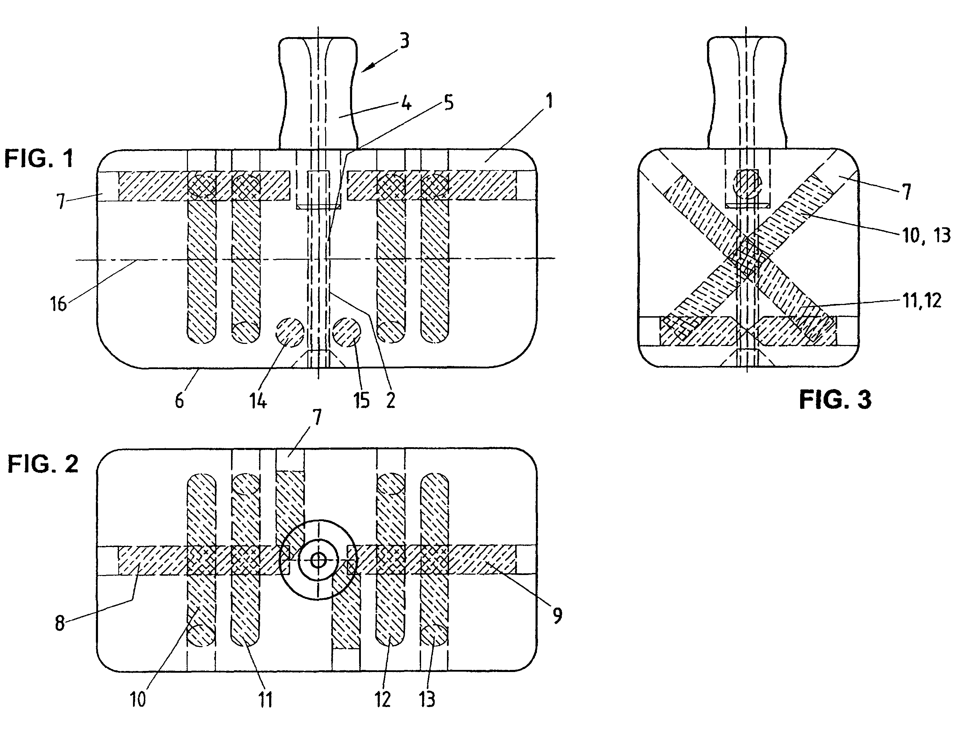

Die Figuren 1 bis 3 zeigen ein Ausführungsbeispiel der erfindungsgemässen Punktiervorrichtung in vergrössertem Massstab. Den Hauptteil der Vorrichtung bildet ein Quader 1 mit abgerundeten Kanten, der vorzugsweise aus Kunststoff besteht. Eine - in Gebrauchslage - von oben nach unten durchgehende Führungsbohrung 2 dient der Aufnahme einer Nadelführung 3, welche aus einem Griff 4 aus Kunststoff und einem damit fest verbundenen Führungsröhrchen 5 aus Titan besteht. Die Nadelführung 3 ist in diesem Ausführungsbeispiel als wegwerfbares Teil konzipiert, wobei der Griff 4 farbig sein kann, damit auf den ersten Blick erkennbar ist, für weichen Nadeldurchmesser die Nadelführung bestimmt ist. Wie in Figur 1 deutlich ersichtlich, reicht das Führungsröhrchen bis zu der auf die Haut des Patienten aufzusetzenden Kontaktfläche 6 des Quaders 1.

Im Quader sind mehrere, in den Figuren 1 bis 3 durch unterbrochene Linien dargestellte Markerbohrungen vorhanden. Sie sind im vorliegenden Beispiel als Sacklochbohrungen ausgeführt, mit einem Kontrastmittel gefüllt und durch Stopfen 7 verschlossen. Als Kontrastmittel können die aus dem Stand der Technik für diesen Zweck bekannten Substanzen verwendet werden. Für MRI-Zwecke hat sich folgende Mischung besonders gut bewährt. lobitridol (oder andere lod-RKM), Dimeglumingadopentetate, Methylhydroxyethylcellulose und Wasser. Der Gadoliniumkonzentratsbereich sollte dabei zwischen 0,5 mM und 40 mM gewählt werden, wobei der optimale Bereich nahe bei 1 mM liegt. Ferner hat sich in der Praxis gezeigt, dass beim CT auch Luft als Kontrastmittel verwendet werden kann. In der senkrechten Längsmittelebene des Quaders befinden sich Markerbohrungen 8 und 9, die von entgegengesetzten Seiten bis in die Nähe der Nadelführung 3 reichen. Weitere Markerbohrungen 10 bis 13 liegen in parallelen, zur genannten senkrechten Längsmittelebene senkrechten Ebenen und ragen von den oberen Kantenbereichen diagonal ins Innere des Quaders. Schliesslich sind noch zwei an ihrem innenliegenden Ende konisch zugespitzte Markerbohrungen 14 und 15 vorgesehen, die von entgegengesetzten Seiten her parallel, zueinander versetzt und in gleichem Abstand von der Kontaktfläche 6 senkrecht bis zur Längsmittelebene reichen. Die sich kreuzenden Bohrungen 10 und 11 beziehungsweise 12 und 13 können sich auch paarweise schneiden.

Im folgenden wird anhand der Zeichnungsfiguren 4 bis 8 dargelegt, wie die oben beschriebene Vorrichtung benutzt wird. Es sei angenommen, der Arzt wolle ein Organ im Bauch eines Patienten mit Hilfe eines MRI Gerätes punktieren. Die erfindungsgemässe Punktiervorrichtung eignet sich sowohl für kurze, geschlossene als auch für offene MRI Systeme. Nachdem sich der Arzt für einen Nadeldurchmesser, beispielsweise 1,2 mm, entschieden hat, wird ein entsprechender, sterilisierter Quader 1 bereitgestellt, dessen Führungsbohrung 2 einen Durchmesser von beispielsweise 1,6 mm hat. Eine Nadelführung 3 wird einer sterilen Verpackung entnommen und in die Führungsbohrung 2 eingesetzt. Um eine Kontamination der Öffnung der Nadelführung zu vermeiden, kann diese verschlossen sein, beispielsweise mit einem Stäbchen aus Kunststoff, welches nach dem Einsetzen der Nadelführung 3 auf der Seite der Kontaktfläche 6 herausgezogen wird.

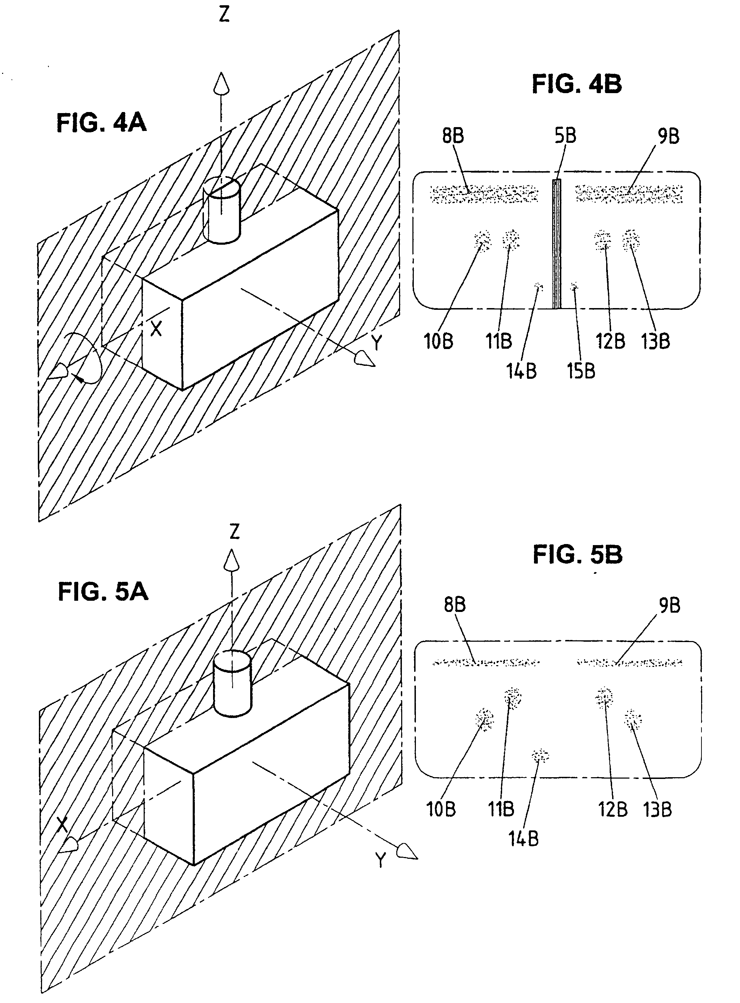

Nachdem das Organ lokalisiert ist, wird die Punktiervorrichtung, das heisst der Quader 1 mit eingesetzter Nadelführung 3, mit der Kontaktfläche 6 auf die Bauchdecke des Patienten aufgesetzt. Sobald der Quader in den Wirkungsbereich des MRI gerät, sind die Markerbohrungen als Schatten auf dem Bildschirm erkennbar. Diese Schatten sind in den Figuren 4B bis 8B mit den Bezugszeichen der entsprechenden Markerbohrungen beziehungsweise des Führungsröhrchens, ergänzt durch ein "B" bezeichnet. Die Punktionsvorrichtung hat die in Figur 4A dargestellte korrekte Ausrichtung erreicht, wenn auf dem Monitor ein Bild entsprechend Figur 4B erscheint. Unter "korrekt" ist in diesem Zusammenhang zu verstehen, dass dann die Nadelführung genau in der Bildebene liegt und somit bei unveränderter Position Gewähr besteht, dass die Punktionsnadel auf ihrem ganzen Weg auf dem Monitor beobachtet werden kann. An dieser Stelle ist noch zu erwähnen, dass insbesondere beim MRI der erfasste Bereich nicht eigentlich eine Ebene ist, sondern eine Schicht mit einer Dicke von 3 bis 6 mm. In Figur 4B ist das aus Titan bestehende Führungsröhrchen 5 der Nadelführung 3 auf seiner ganzen Länge zu erkennen. Die Markerbohrungen 8 und 9 erscheinen als gleichmässig breite Linien 8B und 9B und die Markerbohrungen 10 bis 13 erzeugen ovale Punkte 10B bis 13B, die alle auf einer horizontalen Gerade liegen. Schliesslich sind die in der Nähe der Kontaktfläche 6 liegenden Markerbohrungen 14 und 15 als gleich grosse Punkte 14B und 15B zu erkennen.

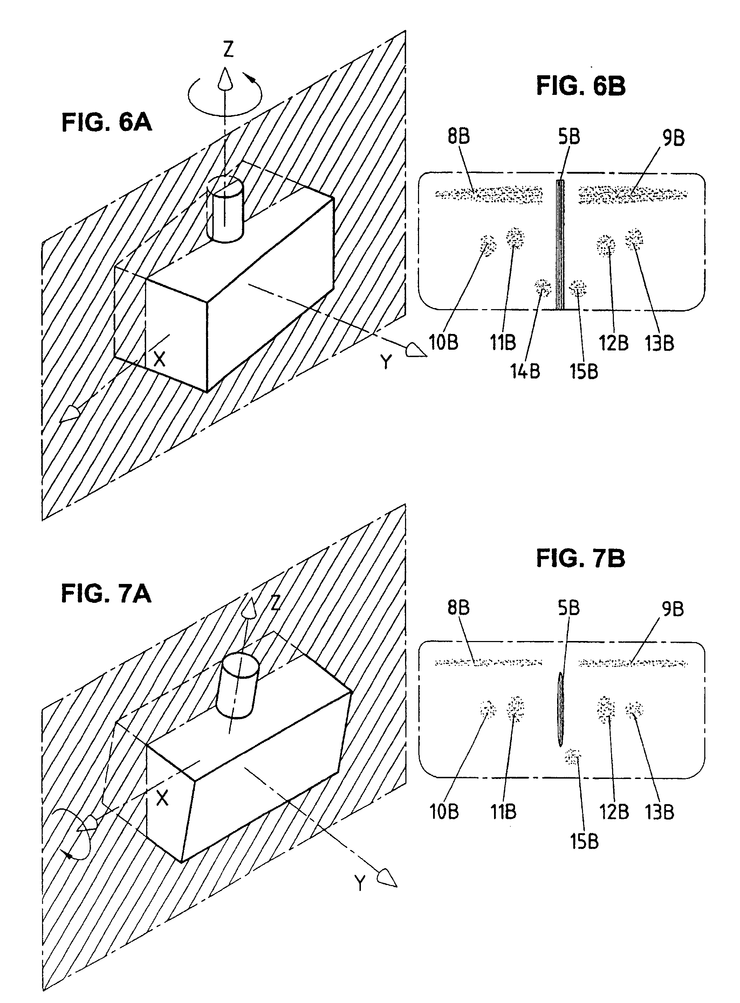

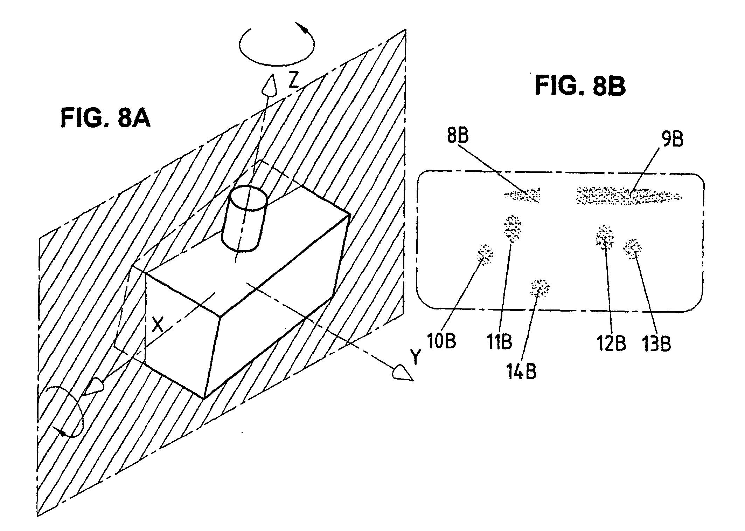

In den Figuren 5 bis 8 sind Ansichten dargestellt, die sich dem Arzt bieten können, wenn die Punktionsvorrichtung bezüglich der Bildebene nicht genau ausgerichtet ist. Figur 5B zeigt ein Bild der in Figur 5A veranschaulichten Lage, bei der die Vorrichtung sich vor der Bildebene befindet. Die Abbilder des Führungsröhrchens 5 sowie der Markerbohrung 15 sind in dieser Figur nicht vorhanden, weil sich diese Teile vor der Bildebene befinden. Aus den schmalen, aber gleichmässig starken Linien 8B und 9B lässt sich ableiten, dass der Quader parallel zur Bildebene ausgerichtet ist. Sowohl an den Ovalen 10B bis 13B, die nicht auf einer Geraden liegen, sondern vertikal gegeneinander versetzt sind, als auch am Fehlen eines Abbildes der Markerbohrung 15, kann man erkennen, dass der Quader vom Betrachter weg verschoben werden muss, damit seine Mittelebene mit der Bildebene ausgerichtet ist. In den Figuren 6A und 6B befindet sich zwar die Nadelführung 3 genau in der Bildebene, jedoch ist die Punktionsvorrichtung um die Achse der Nadelführung verdreht. Dies ist an den nicht gleichmässig starken Linien 8B und 9B zu erkennen, aber auch an den nicht auf einer Geraden ausgerichteten Punkten 10B bis 13B und auch an den Punkten 14B und 15B, deren Durchmesser kleiner sein sollten. Gemäss den Figuren 7A und 7B ist die Punktionsvorrichtung um eine zur Bildebene parallele und zur Nadelführung rechtwinklige Achse X verdreht. Die Punkte 10B bis 13B sind zwar in einer geraden Linie ausgerichtet, woraus sich schliessen lässt, dass der Bereich des Längsmittelachse des Quaders in der Bildebene liegt, jedoch ist deutlich erkennbar, dass nicht alle diese Punkte die gleiche Form besitzen, was auf eine Verdrehung des Quaders um eben diese Längsmittelachse schliessen lässt. Dies ist übrigens auch daran zu erkennen, dass vom Führungsröhrchen 5 nur ein Abbild 5B des mittleren Bereichs sichtbar ist. Schliesslich zeigt das Fehlen des Abbildes der Markerbohrung 14 an, dass der nahe der Kontaktfläche 6 liegende Bereich des Quaders hinter der Bildebene liegt. Dadurch ist dem Arzt auch sofort klar, in welche Richtung die Korrektur erfolgen muss. Schliesslich zeigen die Figuren 8A und 8B noch eine Lage der Punktiervorrichtung, in welcher die in den Figuren 6A, 6B, 7A und 7B dargestellten Fehlstellungen kombiniert sind. Die Lage der Punkte 10B bis 13B zeigt an, dass der Quader links weiter von der korrekten Lage entfernt ist als rechts, wobei am Punkt 14B erkennbar ist, dass der linke Bereich offensichtlich vor der Bildebene liegt. Der Arzt wird folglich zuerst den linken Bereich der Vorrichtung in Richtung zur Bildebene verdrehen, worauf das Bild etwa wie in Figur 7B gezeigt aussehen wird, aus dieser Position genügt dann eine Drehung um die X Achse, wobei der obere Bereich der Vorrichtung gegen die Bildebene gedreht wird, um das in Figur 4B gezeigte Bild und damit eine genaue Ausrichtung der Vorrichtung zu erreichen.

Sobald die Punktionsvorrichtung entsprechend den Figuren 4A und 4B und natürlich auch die zu punktierende Stelle mit der Bildebene ausgerichtet ist, wird die Punktionsnadel in die Nadelführung 3 eingeführt. Dazu hat letztere an ihrem oberen Ende eine konisch erweiterte Öffnung. Bevor der Arzt die Punktionsnadel in den Körper des Patienten einführt, komprimiert er die entsprechende Körperregion, indem er die Punktiervorrichtung gegen die Bauchdecke drückt. Dadurch wird die Gefahr eines Ausweichens des betreffenden Organs während des nachfolgenden Eindringens der Nadel erheblich verringert. Ferner kommt durch diese Massnahme die ganze Vorrichtung näher an das zu punktierende Organ und kann deshalb noch genauer darauf ausgerichtet werden. Da das Führungsröhrchen 5 der Nadelführung 3 bis auf die Haut des Patienten reicht, erfolgt der Einstich genau an der gewünschten Stelle. Dank dem durch die erfindungsgemässe Vorrichtung die Einstichrichtung der Punktionsnadel genau geführt wird, kann deren Weg auf dem Monitor verfolgt und überwacht und die Punktion genau an der gewünschten Stelle vorgenommen werden.

Nach dem Eingriff wird die Nadelführung 3 aus dem Quader 1 entfernt und weggeworfen. Der Quader wird sterilisiert und zur Wiederverwendung bereitgestellt, wobei eine kalte Sterilisationsmethode angewendet werden muss, wenn das Kontrastmittel in den Markerbohrungen flüssig ist.

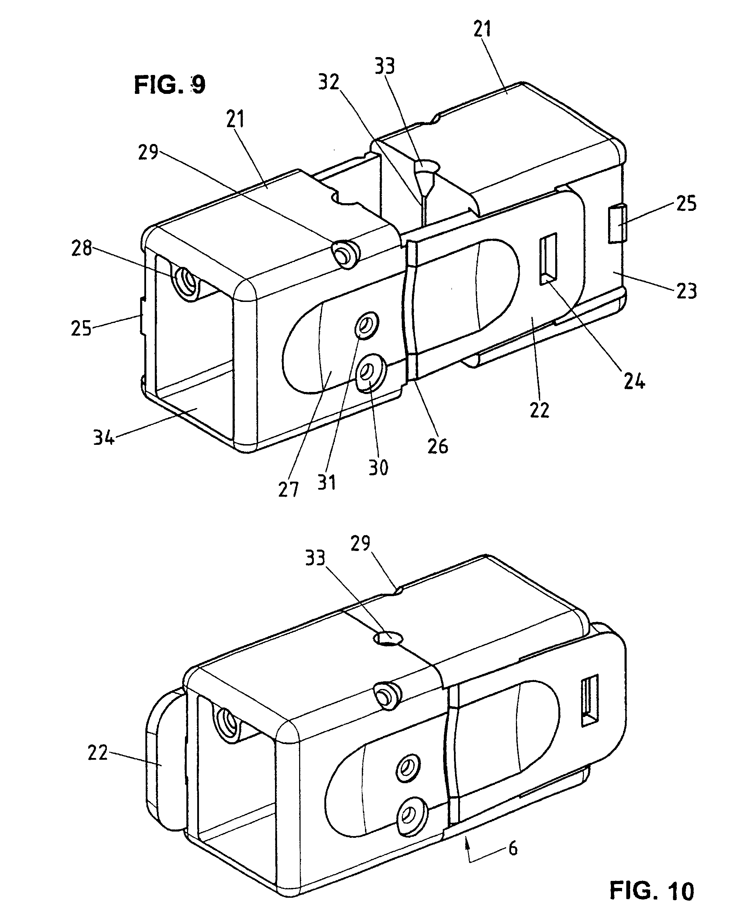

Das in Figur 9 in einer perspektivischen Ansicht gezeigte zweite Ausführungsbeispiel der erfindungsgemässen Punktionsvorrichtung ist zum einmaligen Gebrauch bestimmt und besteht aus zwei identischen, durch Spritzgiessen aus einem Kunststoff hergestellten Teilen 21. Diese Teile 21 sind so gestaltet, dass sie in der in Figur 9 gezeigten Lage zusammengefügt werden können und dann einen Quader bilden, der dem Quader 1 der Figuren 1 bis 3 entspricht. Beim Zusammenfügen der beiden Teile 21 gleitet eine Zunge 22 jedes Teils jeweils in einer am anderen Teil vorgesehene Führung 23, bis die Vorderkante der Zunge durch eine Rampe einer Rastnase 25 leicht angehoben wird. Beim weiteren Zusammenschieben der beiden identischen Teile gerät die Rastnase 25 in den Bereich einer in der Zunge vorgesehenen Ausnehmung 24, so dass die Zunge aus ihrer angehobenen Lage zurückfedert und die Rastnase 25 in der Ausnehmung 24 aufgenommen wird. Diese Endlage, in der die Teile 21 einen dem Quader der Figuren 1 bis 3 entsprechenden Körper bilden, ist in Figur 10 dargestellt. Zur besseren Handhabung des Quaders durch den Arzt ist in den Seitenflächen der Teile 21 eine Griffmulde 27 vorgesehen.

Der durch die Teile 21 gebildete Quader weist ebenfalls Markerbohrungen auf. Dabei entspricht die Markerbohrung 28 den Markerbohrungen 8 und 9 der Ausführungsart nach den Figuren 1 bis 3, die Markerbohrung 29 entspricht den Markerbohrungen 10 und 12 der Figuren 1 bis 3 und die Markerbohrung 30 ist wie die Markerbohrungen 14 und 15 der Figuren 1 bis 3 an ihrem innenliegenden Ende konisch zugespitzt. Die in einer horizontalen Ebene liegende Markerbohrung 31 ersetzt die Markerbohrungen 11 und 13 des Ausführungsbeispiels nach den Figuren 1 bis 3. Die Führungsbohrung 32 hat einen kleineren Durchmesser als die Führungsbohrung 2 nach den Figuren 1 bis 3, weil bei diesem Ausführungsbeispiel auf die Nadelführung 3 verzichtet wird und die Punktionsnadel also direkt im Quader geführt wird. Zu leichteren Einführen der Punktionsnadel ist am oberen Ende der Führungsbohrung 32 eine Ansenkung 33 vorgesehen. Der auf der linken Seite der Figuren 9, 10 und 12 sichtbare Hohlraum 34 ist konstruktionsbedingt, weil bekanntlich Teile, die durch Giessen hergestellt werden, möglichst gleichmässige Wandstärken aufweisen müssen. Für die Funktion der Vorrichtung ist dies aber ohne Bedeutung.

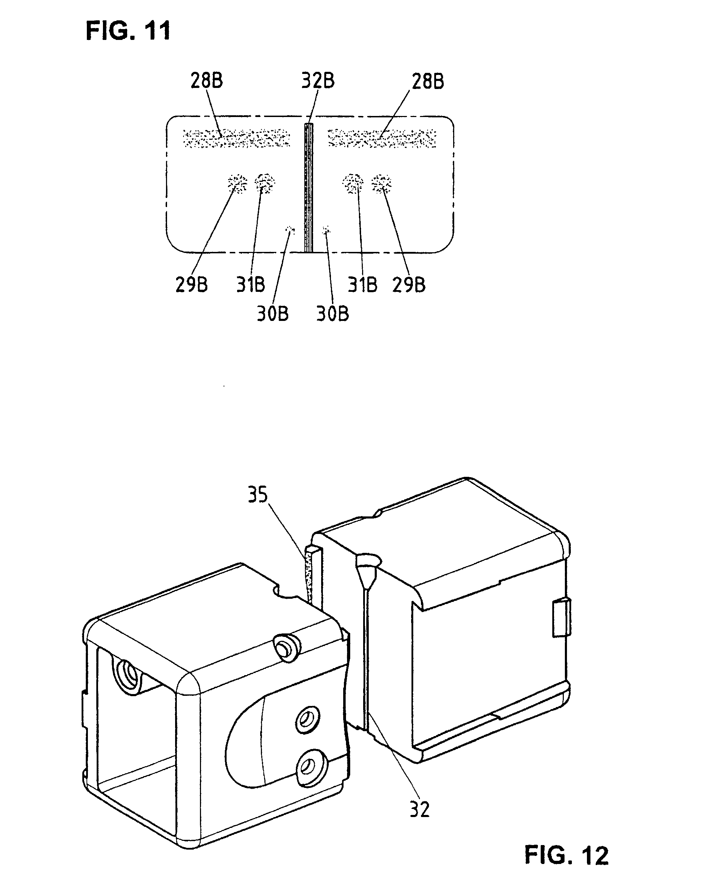

Figur 11 zeigt, ähnlich wie Figur 4B, schematisch eine Schichtaufnahme der Vorrichtung nach Figur 10 bei korrekter Ausrichtung. Die im Quader schräg zur Auflagefläche verlaufenden Markerbohrungen 29 sind als Schatten 29B sichtbar, die sich im Bild nach oben und unten bewegen, wenn der Quader quer zur Bildebene verschoben wird. Die parallel zur Auflagefläche verlaufenden Markerbohrungen 31 erzeugen auf dem Bild die Schatten 31 B, die während einer Bewegung des Quaders quer zur Bildebene am selben Ort bleiben. Somit ist die Punktionsvorrichtung exakt in der Bildebene ausgerichtet, wenn auf dem Bild die Punkte 29B und 31 B längs einer Geraden angeordnet, die Flächen 28B sichtbar und die Punkte 30B gleich gross sind. Damit die durch die geneigt verlaufenden Bohrungen 29 gebildeten Punkte 29B im Bild als rund erscheinen, sind beim vorliegenden Beispiel die Bohrungen 29 in Form einer Ellipse ausgebildet, deren längere Achse parallel zur Grundfläche angeordnet ist.

Wie bereits erwähnt, ist die Punktionsvorrichtung nach den Figuren 9 bis 12 zum einmaligen Gebrauch konzipiert. Zu diesem Zweck wird sie in dem in Figur 10 dargestellten Zustand steril verpackt angeliefert. Sobald die Spitze der Punktionsnadel ihr Ziel im Körper des Patenten erreicht hat, ist die Aufgabe des Quaders erfüllt. Der Quader kann nun den Arzt bei der weiteren Manipulation der Punktionsnadel behindern und es ist deshalb erwünscht, ihn zu entfernen. Zu diesem Zweck besteht der Quader aus den zwei genannten Teilen 21, die nach Gebrauch voneinander trennbar sind. Dazu biegt der Arzt die Zungen 22 nach aussen, um die Ausnehmungen 24 und die Rastnasen 25 ausser Eingriff zu bringen. An der Zunge 22 ist eine Kerbe 26 vorgesehen, welche bewirkt, dass die Zunge abbricht, wenn sie nach aussen gebogen wird. Dadurch wird ein Wiederverwenden der nicht mehr sterilen Vorrichtung sicher verhindert. Figur 12 zeigt die beiden getrennten Teile mit der im Bereich der Kerbe entstandenen Bruchfläche 35. Die Zunge 22 und die Kerbe 26 sind so dimensioniert, dass die Zunge beim Montieren der beiden Teile 21 nicht bricht. Gegebenenfalls können die Teile zum Montieren leicht erwärmt werden.

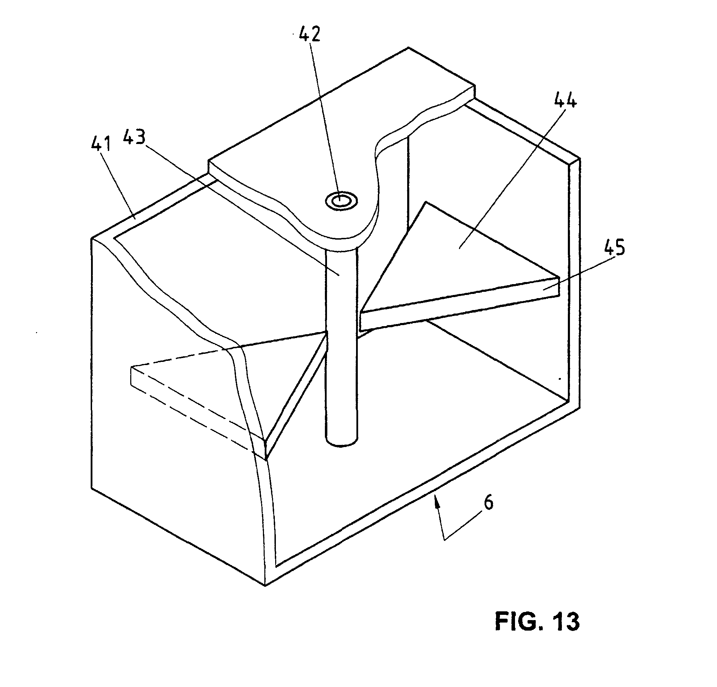

In Figur 13 ist eine weitere, alternative Ausgestaltung der erfindungsgemässen Punktionsvorrichtung perspektivisch und teilweise aufgeschnitten dargestellt. Das vorangehend beschriebene Prinzip ist hier umgekehrt, indem der Quader 41 im wesentlichen hohl ausgebildet und mit Kontrastmittel gefüllt ist. Die Führungsbohrung 42 für die Punktionsnadel befindet sich in.einem Führungsröhrchen 43, das den hohlen Quader durchsetzt. Mit beiden stimseitigen Wänden des Quaders ist je ein Richtkörper 44 verbunden, der zum Ausrichten der Vorrichtung mit der Bildebene dient. Jeder Richtkörper 44 verjüngt sich in Richtung auf die Führungsbohrung, so dass seine seitlichen Begrenzungsflächen 45 länglich ausgebildet und nicht parallel zu den Führungsmitteln und nicht parallel zueinander angeordnet sind. Dadurch ergibt sich im Betrieb ein ähnlicher Effekt ergibt wie bei den vorher beschriebenen Ausführungsarten. Je mehr sich die Mittelebene des Quaders, in welcher sich die Führungsbohrung 42 befindet, der Bildebene nähert, desto länger werden die durch die Richtkörper 44 gebildeten Schatten, die sich von den Stirnflächen des Quaders in Richtung auf die Führungsbohrung erstrecken. Selbstverständlich können auch bei dieser Ausführungsart weitere Richtkörper vorgesehen sein, die das genaue Positionieren des Quaders in der Aufnahmeebene unterstützen. Diese Richtkörper können so angeordnet sein, wie die Markerbohrungen der vorangehend beschriebenen Ausführungsbeispiele, nur dass es sich hier nicht um Bohrungen handelt, sondern um Körper, die sich von der Wänden des hohlen Quaders in dessen Inneres erstrecken.

## Patentansprüche

1. Punktionsvorrichtung für Schichtaufnahmeverfahren, enthaltend einen Körper, mit der Aufnahmeebene auszurichtende Führungsmittel für eine Punktionsnadel und im Körper angeordnete, längliche Hohlräume, die ein Kontrastmittel enthalten, **dadurch gekennzeichnet, dass** der Körper im wesentlichen die Form eines Quaders (1; 21) hat, wobei dessen zur Längsachse (16) parallele Grundfläche (6) zum Aufsetzen auf die Haut eines Patienten bestimmt ist, dass die Achse der Führungsmittel (2, 3; 32) die Grundfläche (6) in einem rechten Winkel schneidet und dass mindestens zwei (10, 11; 12, 13; 29) der länglichen Hohlräume rechtwinklig zur Längsachse, aber nicht parallel zu den Führungsmitteln und nicht parallel zueinander angeordnet sind.

2. Punktionsvorrichtung nach Anspruch 1, **dadurch gekennzeichnet, dass**, in bezug auf die Längsachse (16) des Quaders, beiderseits der Führungsmittel (2, 3) je zwei der länglichen Hohlräume rechtwinklig zur Längsachse, aber nicht parallel zu den Führungsmitteln und nicht parallel zueinander angeordnet sind.

3. Punktionsvorrichtung nach Anspruch 1 oder 2, **dadurch gekennzeichnet, dass** in der durch die Längsachse (16) und die Achse der Führungsmittel definierten Ebene je ein länglicher Hohlraum (8, 9; 28) von entgegengesetzten Stirnseiten des Quaders bis in die Nähe der Führungsmittel reicht.

4. Punktionsvorrichtung nach einem der vorangehenden Ansprüche, **dadurch gekennzeichnet, dass** rechtwinklig zu der durch die Längsachse (16) und die Achse der Führungsmittel definierten Ebene je ein länglicher Hohlraum (14, 15; 30) von entgegengesetzten Seiten des Quaders bis zu dieser Ebene reicht, wobei die bis zur genannten Ebene reichenden Enden dieser Hohlräume vorzugsweise zugespitzt sind.

5. Punktionsvorrichtung nach einem der vorangehenden Ansprüche, **dadurch gekennzeichnet, dass** die länglichen Hohlräume als Sacklochbohrungen ausgeführt sind, deren aussen liegendes Ende (7) verschlossen ist.

6. Punktionsvorrichtung nach einem der vorangehenden Ansprüche, **dadurch gekennzeichnet, dass** die Führungsmittel aus einem in eine Führungsbohrung (2) des Quaders (1) wegnehmbar eingesetzten Führungsröhrchen (5) bestehen, an dessen einem Ende ein Griff (4) mit einer konischen Einführungsöffnung für eine Punktionsnadel befestigt ist.

7. Punktionsvorrichtung nach einem der vorangehenden Ansprüche, **dadurch gekennzeichnet, dass** der Quader im Bereich der Führungsmittel in mindestens zwei Teile (21) trennbar ist, derart dass diese mindestens zwei Teile von der Punktionsnadel entfernbar sind, ohne dass die Punktionsnadel längs ihrer Achse zurückgezogen wird.

8. Punktionsvorrichtung nach Anspruch 7, **dadurch gekennzeichnet, dass** der Quader aus mindestens zwei Teilen (21) zusammengesetzt ist, die in der Ebene der Führungsmittel aneinanderstossen.

9. Punktionsvorrichtung nach Anspruch 8, **dadurch gekennzeichnet, dass** die mindestens.zwei Teile (21) durch Rastmittel enthaltende Teile (22, 23, 24, 25) zusammengehalten werden.

10. Punktionsvorrichtung nach Anspruch 9, **dadurch gekennzeichnet, dass** die Teile federnde Zungen (22) sind, die an mindestens einem der Teile (21) angeordnet sind und in Führungen (23) eines anderen Teils (21) eingreifen und dass die Rastmittel eine Rastnase (25) umfassen.

11. Punktionsvorrichtung nach Anspruch 10, **dadurch gekennzeichnet, dass** an den federnden Zungen (22) eine Sollbruchstelle (26) vorhanden ist, die bricht, wenn die Zunge zwecks Freigabe der Rastnase gebogen wird.

12. Punktionsvorrichtung nach einem der Ansprüche 1 bis 5 und 7 bis 11, **dadurch gekennzeichnet, dass** die Führungsmittel aus einer Führungsbohrung (32) des Quaders bestehen, die an dem von der Grundfläche abgewandten Ende eine konische Erweiterung (33) zur leichteren Einführung der Punktionsnadel aufweist.

13. Punktionsvorrichtung für Schichtaufnahmeverfahren, enthaltend einen Hohlkörper, der ein Kontrastmittel enthält, mit der Aufnahmeebene auszurichtende Führungsmittel für eine Punktionsnadel und im Hohlkörper angeordnete Richtkörper, **dadurch gekennzeichnet, dass** der Hohlkörper im wesentlichen die Form eines Quaders (41) hat, wobei dessen zur Längsachse parallele Grundfläche (6) zum Aufsetzen auf die Haut eines Patienten bestimmt ist, dass die Achse der Führungsmittel (42) die Grundfläche (6) in einem rechten Winkel schneidet und dass die Richtkörper längliche Begrenzungsflächen (45) aufweisen, die nicht parallel zu den Führungsmitteln und nicht parallel zueinander angeordnet sind.

## Claims

1. Puncturing device for tomography, containing a body, with guide means, to be aligned with the picture plane, for a puncture needle, and elongated hollow spaces, disposed in the body, which contain contrast medium, **characterised in that** the body has substantially the shape of a cuboid (1; 21), its base area (6) parallel to the longitudinal axis (16) being intended for placement on the skin of a patient, **in that** the axis of the guide means (2, 3; 32) cuts the base area (6) at a right angle, and **in that** at least two (10, 11; 12, 13; 29) of the elongated hollow spaces are disposed at a right angle to the longitudinal axis, but not parallel to the guide means and not parallel to one another.

2. Puncturing device according to claim 1, **characterised in that**, in relation to the longitudinal axis (16) of the cuboid, on both sides of the guide means (2, 3), two each of the elongated hollow spaces are disposed at a right angle to the longitudinal axis, but not parallel to the guide means and not parallel to one another.

3. Puncturing device according to claim 1 or 2, **characterised in that** in the plane defined by the longitudinal axis (16) and the axis of the guide means, one elongated hollow space (8, 9; 28) each extends from opposite end faces of the cuboid into the vicinity of the guide means.

4. Puncturing device according to one of the preceding claims, **characterised in that** at a right angle to the plane defined by the longitudinal axis (16) and the axis of the guide means one elongated hollow space (14, 15; 30) each extends from opposite sides of the cuboid up to this plane, the ends of these hollow spaces extending up to said plane being preferably pointed.

5. Puncturing device according to one of the preceding claims, **characterised in that** the elongated hollow spaces are designed as blind bores whose outer-lying end (7) is closed.

6. Puncturing device according to one of the preceding claims, **characterised in that** the guide means consist of a guide tube (5) removably inserted in a guide bore (2) of the cuboid (1), on the one end of which tube is fastened a handle (4) with a conical insertion opening for a puncture needle.

7. Puncturing device according to one of the preceding claims, **characterised in that** the cuboid is separable into at least two parts (21) in the region of the guide means such that these at least two parts are removable from the puncture needle without the puncture needle having to be pulled back along its axis.

8. Puncturing device according to claim 7, **characterised in that** the cuboid is composed of at least two parts (21) which meet in the plane of the guide means.

9. Puncturing device according to claim 8, **characterised in that** the at least two parts (21) are held together through parts (22, 23, 24, 25) containing engagement means.

10. Puncturing device according to daim 9, **characterised in that** the parts are resilient tabs (22) which are disposed on at least one of the parts (21) and engage in guides (23) of another part (21), and **in that** the engagement means comprise a locking projection (25).

11. Puncturing device according to claim 10, **characterised in that** a break-off site (26) is disposed on the resilient tabs (22), which breaks if the tabs are bent for the purpose of releasing the locking projection.

12. Puncturing device according to one of the claims 1 to 5 and 7 to 11, **characterised in that** the guide means consist of a guide bore (32) of the cuboid, which has on the end remote from the base area a conical extension (33) for easier insertion of the puncture needle.

13. Puncturing device for tomography, comprising a hollow body, which contains a contrast medium, guide means, to be aligned with the picture plane, for a puncture needle, and alignment bodies disposed in the hollow body, **characterised in that** the hollow body has substantially the shape of a cuboid (41), its base area (6) parallel to the longitudinal axis being intended for placement on the skin of a patient, **in that** the axis of the guide means (42) cut the base area (6) at a right angle, and **in that** the alignment bodies have elongated peripheries (45), which are disposed not parallel to the guide means and not parallel to one another.

## Revendications

1. Dispositif de ponction pour un procédé de tomographie, comportant un corps, des moyens de guidage à ajuster au plan de prise pour une aiguille de ponction et des cavités allongées disposées dans le corps, qui contiennent un moyen de contraste, **caractérisé en ce que** le corps a sensiblement la forme d'un parallélépipède (1; 21), dont la surface de base(6) parallèle à l'axe longitudinal (16) est destinée à être placée sur la peau d'un patient, **en ce que** l'axe des moyens de guidage (2, 3; 32) coupe la surface de base (6) en formant un angle droit et **en ce qu'**au moins deux (10, 11; 12, 13; 29) des cavités allongées sont disposées en angle droit par rapport à l'angle longitudinal mais ne sont pas parallèles aux moyens de guidage et ne sont pas parallèles entre elles.

2. Dispositif de ponction selon la revendication 1, **caractérisé en ce que** par rapport à l'axe longitudinal (16) du parallélépipède, de part et d'autre des moyens de guidage (2, 3), respectivement deux des cavités allongées sont disposées en angle droit par rapport à l'axe longitudinal mais pas parallèles aux moyens de guidage et pas parallèles entre elles.

3. Dispositif de ponction selon la revendication 1 ou 2, **caractérisé en ce que** dans le plan défini par l'axe longitudinal (16) et l'axe des moyens de guidage, une cavité allongée (8, 9; 28) s'étend des faces frontales opposées du parallélépipède jusqu'à la proximité des moyens de guidage.

4. Dispositif de ponction selon l'une des revendications précédentes **caractérisé en ce qu'**en formant un angle droit avec le plan défini par l'axe longitudinal (16) et l'axe des moyens de guidage, une cavité allongée (14, 15; 30) s'étend des faces opposées du parallélépipède jusqu'à ce plan, les extrémités de ces cavités qui s'étendent jusqu'à ce plan étant pointues.

5. Dispositif de ponction selon l'une des revendications précédentes, **caractérisé en ce que** les cavités allongées sont réalisées comme des trous borgnes dont l'extrémité extérieure (7) est fermée.

6. Dispositif de ponction selon l'une des revendications précédentes, **caractérisé en ce que** les moyens de guidage se composent d'un petit tube de guidage (5) inséré de manière amovible dans un trou de guidage (2) du parallélépipède (1) et à l'extrémité duquel est fixée une poignée (4) avec une ouverture d'insertion conique pour une aiguille de ponction.

7. Dispositif de ponction selon l'une des revendications précédentes, **caractérisé en ce que** le parallélépipède est séparable dans la zone des moyens de guidage en au moins deux parties (21) de sorte que ces deux parties peuvent être retirées de l'aiguille de ponction sans avoir à retirer l'aiguille de ponction le long de son axe.

8. Dispositif de ponction selon la revendication 7, **caractérisé en ce que** le parallélépipède est composé d'au moins deux parties (21) qui sont aboutées dans le plan des moyens de guidage.

9. Dispositif de ponction selon la revendication 8, **caractérisé en ce qu'**au moins les deux parties (21) sont maintenues l'une à l'autre par des pièces (22, 23, 24, 25) contenant des moyens d'encliquetage.

10. Dispositif de ponction selon la revendication 7, **caractérisé en ce que** les pièces sont des languettes élastiques (22) qui sont disposées sur au moins une des pièces (21) et s'engagent dans des guides (23) d'une autre pièce (21) et **en ce que** les moyens d'encliquetage présentent un cran d'arrêt (25).

11. Dispositif de ponction selon la revendication 10, **caractérisé en ce qu'**il est prévu sur les languettes élastiques (22) une position de rupture (26) qui casse quand la languette est fléchie pour libérer le cran d'arrêt (25).

12. Dispositif de ponction selon l'une des revendications 1 à 5 et 7 à 11, **caractérisé en ce que** les moyens guidage se composent d'un trou de guidage (32) du parallélépipède, trou qui présente sur l'extrémité détournée de la surface de base un élargissement conique (33) pour une introduire plus facilement l'aiguille de ponction.

13. Dispositif de ponction pour un procédé de tomographie, comportant un corps creux qui contient un produit de contraste, des moyens de guidage à ajuster au plan de prise pour une aiguille de ponction et des corps d'ajustement disposés dans le corps creux, **caractérisé en ce que** le corps creux a sensiblement la forme d'un parallélépipède (41), dont la surface de base (6) parallèle à l'axe longitudinal est destinée à être placée sur la peau d'un patient et **en ce que** l'axe des moyens de guidage (42) coupe la surface de base (6) en formant un angle droit et **en ce que** les corps d'ajustement présentent des surfaces allongées de délimitation (45) qui ne sont pas disposées parallèlement aux moyens de guidage et pas parallèles entre elles.
